# EUROPÄISCHE PATENTSCHRIFT

(11) **EP 1 970 016 B1**
(45) Veröffentlichungstag und Bekanntmachung des Hinweises auf die Patenterteilung: **21.07.2010**
(21) Anmeldenummer: 08004730.1
(22) Anmeldetag: 13.03.2008
(51) Int. Cl.: A61B 17/16

(54) **Vorrichtung zum Befestigen eines chirurgischen Fadens an einem Knochen**
Device for securing a surgical thread to a bone
Dispositif de fixation d'un fil chirurgical sur un os

(30) Priorität: 13.03.2007 DE 102007013426
(43) Veröffentlichungstag der Anmeldung: 17.09.2008
(73) Patentinhaber: Karl Storz GmbH & Co. KG, 78532 Tuttlingen (DE)
(72) Erfinder: Sauer, Michael, 78532 Tuttlingen (DE); Frey, Sebastian, 78054 Villingen-Schwenningen (DE)
(74) Vertreter: Weller, Wolfgang

(56) Entgegenhaltungen:
- WO-A-00/74578
- US-A- 5 522 820
- US-A1- 2003 078 599

## Beschreibung

Die Erfindung betrifft eine Vorrichtung zum Befestigen eines chirurgischen Fadens an einem Knochen, mit einem ersten Element, das in einen Knochen einbringbar ist, und mit einem zweiten bewegbaren Element, das derart in den Knochen einbringbar ist, dass es das erste Element trifft, wodurch ein Kanal in dem Knochen bewerkstelligt wird, wobei eines der Elemente einen chirurgischen Faden derart trägt, dass der Faden, nachdem die Elemente sich getroffen haben, ergreifbar ist.

Eine derartige Vorrichtung ist aus der US 6,843,796 B2 und der WO 00/74578 A2 bekannt.

Mittels einer solchen Vorrichtung kann ein bogenförmiger Kanal in einem Knochen bewerkstelligt werden. Danach wird die Vorrichtung von dem Knochen abgenommen und durch den Kanal wird ein chirurgischer Faden mit Hilfe eines Fadenschiebers durchgeführt. Mit den freien Enden des aus den beiden Enden des Kanals vorstehenden Fadens kann ein Knochenstück, abgerissenes Gewebe oder ein ein Implantat haltender Faden an dem Knochen fixiert werden.

Die Vorrichtung der US 6,843,796 B2 weist zwei aufeinander zu bewegbare gekrümmte Elemente auf. Durch jedes Element geht ein Kanal hindurch. Die Elemente sind wie die Backen einer Beißzange geformt. An einem distalen Ende eines der Elemente ist eine spitze Sonde angeordnet, an der ein chirurgischer Faden fixiert ist. Somit wird der chirurgische Faden von diesem bewegbaren Element in den Knochen eingebracht.

Darüber hinaus wird in der WO 00/74578 A2 eine Vorrichtung beschrieben, in der zunächst zwei feststehende Elemente senkrecht in den Knochen eingetrieben werden und ein weiteres bewegbares gekrümmtes Element aus dem einen feststehenden Element schräg auf das zweite feststehende Element zubewegt wird. Dabei ist der chirurgische Faden am distalen Ende dieses bewegbaren gekrümmten Elementes entsprechend fixiert, wodurch der chirurgische Faden in analoger Weise zu dem zuvor Beschriebenen in den Knochen eingebracht wird.

Beim Bewerkstelligen des Kanals werden die bewegbaren Elemente/das bewegbare Element in den Knochen eingetrieben, und zwar so weit, bis die Sonde, an der der Faden fixiert ist, auf ein distales Ende des zweiten Elements im Inneren des Knochens trifft. Dabei fährt die Sonde etwas in den Kanal des zweiten Elements hinein. Beim Zurückziehen des zweiten Elements soll die Sonde in diesem stecken bleiben und der Faden soll samt Sonde mittels des zweiten Elements aus dem Knochen herausgezogen werden.

In der WO 00/74578 A2 ist hierzu ein Schlauch mit einem Schlitz in dem einen Element vorgesehen, in dem die Sonde eingeführt wird und einhakt.

Bei diesen Vorrichtungen stellt sich als nachteilig heraus, dass sich die Sonde beim Zurückziehen des zweiten Elements von diesem lösen kann und in dem Kanal verbleibt. Dann muss das zweite Element nochmals eingetrieben werden, um die verlorene Sonde wieder einzufangen. Es kann auch vorkommen, dass die Übergabe der Sonde von dem ersten Element zu dem zweiten Element nicht funktioniert, dass diese also an dem ersten Element verbleibt. Die Fehlfunktion wird erst bemerkt, wenn das zweite Element von dem Knochen abgezogen ist, denn der Übergabeort liegt im Inneren des Knochens, und er ist somit von außen nicht ersichtlich. Das ggf. mehrfache Eintreiben der Elemente führt zu einer unnötigen Traumatisierung.

Es ist daher Aufgabe der vorliegenden Erfindung, eine Vorrichtung der eingangs genannten Art dahingehend weiter zu entwickeln, dass die Fadenübergabe bzw. Fadenübernahme mit hoher, möglichst 100%iger Erfolgsrate durchführbar ist.

Erfindungsgemäß wird die Aufgabe dadurch gelöst, dass in dem Element, das keinen Faden trägt, ein separater Fadenfänger angeordnet ist, mittels dem der Faden, nachdem die Elemente sich getroffen haben, ergreifbar und über dieses Element abziehbar ist, wobei die beiden Elemente beim Abziehen des Fadens im Knochen in der Lage bleiben, in der sie sich getroffen haben.

Es ist erfindungsgemäß vorgesehen, dass in dem Element, das keinen Faden trägt, ein separater Fadenfänger angeordnet ist. Dadurch wird erreicht, dass der Faden, nachdem die beiden Elemente sich getroffen haben, von dem Fadenfänger ergriffen werden kann und über das Element, in dem der Fadenfänger angeordnet ist, abgezogen werden kann, ohne dass die Gefahr besteht, dass sich der Faden beim Abziehen von dem Fadenfänger löst.

Die beiden Elemente bleiben im Knochen in der Lage, in der sie sich getroffen haben. Die Handhabungsperson ergreift mit dem Fadenfänger den Faden, der von dem den Faden tragenden Element an das den Fadenfänger aufweisende Element herangeführt worden ist. Das den Faden tragende Element trägt diesen so ausgerichtet, dass dieser von dem Fadenfänger ergriffen wird, wenn dieser gehandhabt wird. Das Element, das den Fadenfänger aufweist, führt diesen zwangsweise in eine Position, in der der Faden von dem Fadenfänger gefangen wird. Jetzt kann der Faden mittels des Fadenfängers über das erste Element von dem Knochen abgezogen werden. Diese Übergabe bzw. Übernahme des Fadens kann mit hoher, nahezu 100%iger Erfolgsrate durchgeführt werden.

Bei einer ersten prinzipiellen Vorgehensweise ist der Fadenfänger in dem ersten Element angeordnet und der Faden wird von dem zweiten Element getragen. Zunächst wird das erste Element in den Knochen eingetrieben. Danach wird das zweite, bewegbare Element, das den chirurgischen Faden trägt, in ein im Knochen vorhandenes Loch so weit eingetrieben, dass es das erste Element trifft. Nachdem die beiden Elemente sich getroffen haben, wird der Faden vom dem Fadenfänger, der in dem ersten Element angeordnet ist, ergriffen und über das erste Element abgezogen. Erst danach, also wenn die freien Enden des Fadens außerhalb des Knochens vom Operateur festgehalten werden, werden die beiden Elemente der Vorrichtung von dem Knochen abgezogen.

Bei einer zweiten prinzipiellen Vorgehensweise ist der Faden von dem ersten Element getragen und der Fadenfänger ist dagegen in dem zweiten Element angeordnet. Zunächst wird das erste, den Faden tragende Element in den Knochen eingetrieben. Das zweite Element wird ebenfalls in den Knochen eingetrieben, und zwar so weit, bis es das erste Element trifft. In diesem Zustand wird der Faden von dem in dem zweiten Element angeordneten Fadenfänger ergriffen und durch das zweite bewegbare Element abgezogen.

In einer Ausgestaltung der Erfindung ist das erste Element unbewegbar und stabförmig ausgebildet.

Diese Maßnahme hat den Vorteil, dass beim Ansetzen der erfindungsgemäßen Vorrichtung an die gewünschte Stelle an dem Knochen dieses unbewegbar ausgebildete erste Element, das vorteilhafterweise zuerst in den Knochen eingetrieben wird, einen ortsfesten Ansatzpunkt definiert. Das derartig positionierte unbewegbare Element, das stabförmig ausgebildet ist, kann von dem Operateur einfach in den Knochen eingetrieben werden.

In einer weiteren Ausgestaltung der Erfindung ist ein distales Ende des ersten Elements als Spitze ausgebildet.

Hierbei ist von Vorteil, dass ein stabförmiges Element, dessen distales Ende als Spitze ausgebildet ist, einfach und wenig traumatisch in den Knochen eingetrieben werden kann.

In einer weiteren Ausgestaltung der Erfindung ist der Fadenfänger in dem ersten, stabförmigen Element angeordnet.

Diese Maßnahme hat den Vorteil, dass der Fadenfänger, ebenfalls wie das erste Element, einen geraden Körper aufweist, der vorzugsweise aus einem starren Material ausgebildet ist. Dies trägt dazu bei, dass der Faden, nachdem die beiden Elemente sich getroffen haben, von dem derartig ausgebildeten Fadenfänger durch eine geradlinige Verschiebebewegung einfach ergriffen werden kann.

In einer weiteren Ausgestaltung der Erfindung ist ein distaler Abschnitt des ersten Elements mit einer Öffnung versehen.

Diese Maßnahme hat den Vorteil, dass durch diese Öffnung das zweite Element in das erste hineinfahren kann. Dadurch wird ein Kreuzungspunkt von erstem Element und zweitem Element, nachdem die beiden Elemente sich getroffen haben, geschaffen. Der Kreuzungspunkt definiert eine Übergabestation des Fadens. Durch eine derartige Ausgestaltung des ersten Elements wird ermöglicht, dass der Faden an eine Übergabestation gebracht wird, an der er von dem in dem ersten Element angeordneten Fadenfänger ergriffen werden kann. Dies trägt ebenfalls dazu bei, dass die Fadenübergabe bzw. -übernahme mit hoher Erfolgsrate durchführbar ist.

In einer weiteren Ausgestaltung der Erfindung ist dazu ein distaler, den Faden tragender Abschnitt des zweiten Elements in die Öffnung in dem ersten Element einfahrbar.

Durch eine derartige Ausgestaltung der erfindungsgemäßen Vorrichtung wird sichergestellt, dass der Faden, der von dem distalen Abschnitt des zweites Elements getragen wird, durch Einfahren des zweiten Elements in die Öffnung in dem ersten Element an die Übergabestation gebracht wird. Somit kann der Fadenfänger, der in dem ersten, stabförmigen Element angeordnet ist, den sich an der Übergabestation befindlichen Faden sicher ergreifen.

In einer weiteren Ausgestaltung der Erfindung trägt das zweite Element an seinem distalen Abschnitt den Faden derart, dass ein Fadenabschnitt des Fadens freiliegend ist, der, nachdem die Elemente sich getroffen haben, von dem in dem ersten Element angeordneten Fadenfänger zwangsweise ergreifbar ist.

Durch eine derartige Ausgestaltung der erfindungsgemäßen Vorrichtung hängt das Ergreifen bzw. Fangen des freiliegenden Fadenabschnitts des Fadens mit dem Fadenfänger nicht von der Geschicklichkeit oder von der Aufmerksamkeit der Person ab, die die Vorrichtung bedient, da der Fadenfänger den an die Übergabestation gebrachten Faden zwangsweise ergreift. Dies führt zur Vermeidung von Fehlern beim Ergreifen des Fadens. Somit kann die Fadenübernahme bzw. -übergabe mit 100 %iger Erfolgsrate durchgeführt werden.

In einer weiteren Ausgestaltung der Erfindung ist der Fadenfänger nur in einer definierten Position in das erste Element einbringbar.

Hierbei besteht der Vorteil, dass das lagegerechte Einführen des Fadenfängers in das erste Element nicht von der Geschicklichkeit des Operateurs abhängig ist, weil das richtige Einführen durch die Ausgestaltung der erfindungsgemäßen Vorrichtung vorgegeben ist. Mit anderen Worten kann der Operateur den Fadenfänger in das erste Element nur so einführen, dass der Fadenfänger den an die Übergabestation gebrachten Faden zwangsweise ergreift.

In einer weiteren Ausgestaltung der Erfindung ist in dem ersten Element ein Hohlraum zur Aufnahme des Fadenfängers vorhanden.

Dadurch, dass der Fadenfänger in dem ersten Element aufgenommen ist, kann der Fadenfänger zusammen mit dem ersten Element in den Knochen eingetrieben werden, ohne dass die Gefahr besteht, dass der Fadenfänger beschädigt wird. Ferner kann zunächst das erste Element in den Knochen eingetrieben werden und danach der Fadenfänger in den Hohlraum in dem ersten Element eingeführt werden.

Darüber hinaus bietet der Hohlraum in dem ersten Element eine zielgerichtete Führung für den Fadenfänger, so dass der Fadenfänger zur Übergabestation hingeführt wird, wo er den Faden sicher ergreift.

In einer weiteren Ausgestaltung der Erfindung mündet der Hohlraum an seinem distalen Ende in die Öffnung in dem ersten Element.

Diese Maßnahme hat den Vorteil, dass der Fadenfänger durch den Hohlraum in die an dem distalen Abschnitt des ersten Elements angeordneten Öffnung, die die Übergabestation definiert, hineinragen kann. Somit wird sichergestellt, dass der Fadenfänger den freiliegenden Fadenabschnitt des Fadens, nachdem der distale Abschnitt des zweiten Elements in die Öffnung in dem ersten Element eingefahren ist, sicher ergreift.

In einer weiteren Ausgestaltung der Erfindung weist der den Faden tragende distale Abschnitt des zweiten Elements eine Ausnehmung auf, über die der freiliegende Fadenabschnitt von dem Fadenfänger ergreifbar ist.

Auch hierbei besteht wiederum der Vorteil, dass der freiliegende Fadenabschnitt des Fadens über die Ausnehmung von dem Fadenfänger einfach und sicher ergriffen werden kann.

In einer weiteren Ausgestaltung der Erfindung weist der distale Abschnitt des zweiten Elements eine Bohrung zum Durchführen des Fadens auf, die die Ausnehmung schneidet.

Diese Maßnahme hat den Vorteil, dass der freiliegende Fadenabschnitt quer zu der Ausnehmung in dem zweiten Element angeordnet ist, so dass der freiliegende Fadenabschnitt des Fadens über die Ausnehmung mit dem Fadenfänger ergriffen werden kann.

In einer weiteren Ausgestaltung der Erfindung ist die Ausnehmung als eine Nut ausgebildet.

Diese Maßnahme hat den Vorteil, dass die Nut fertigungstechnisch besonders einfach in das zweite Element eingebracht werden kann, wodurch der Herstellungsaufwand der Vorrichtung bei dieser Ausgestaltung verringert ist.

Alternativ dazu ist es bevorzugt, die Ausnehmung als Langloch auszubilden.

Diese Ausgestaltung der Ausnehmung in Form eines Langlochs hat den Vorteil, dass der freiliegende Fadenabschnitt des Fadens durch die Ausnehmung einfach ergreifbar ist. Der Fadenfänger kann zunächst durch das Langloch hindurchgeführt und dann hinter den Fadenabschnitt gebracht werden, um diesen dann nach oben abzuziehen.

In einer weiteren Ausgestaltung der Erfindung ist das zweite Element gekrümmt.

Diese Maßnahme hat den Vorteil, dass durch eine derartige Kombination eines stabförmigen Elements mit einem gekrümmten Element eine Vorrichtung geschaffen wird, mit der ein Kanal in dem Knochen bewerkstelligt wird, der etwa viertelkreisförmig ausgebildet ist. Somit ist dieser Kanal kürzer als ein etwa halbkreisförmiger Kanal, der mit der bekannten Vorrichtung bewerkstelligt wird. Dies führt dazu, dass der Knochen weniger beeinträchtigt wird.

In einer weiteren Ausgestaltung der Erfindung weist das zweite Element an seinem distalen Ende eine Spitze auf.

Auch hierbei besteht wiederum der Vorteil, dass das gekrümmte Element einfach und wenig traumatisch in ein in dem Knochen vorhandenes Loch eingetrieben werden kann.

In einer weiteren Ausgestaltung der Erfindung ist das zweite Element entlang einer Führungsbahn führbar.

Diese Maßnahme hat den Vorteil, dass das zweite Element längst einer fest vorgegebenen Wegstrecke führbar ist, die so ausgerichtet ist, dass der distale Abschnitt des zweiten Elements in die Öffnung in dem ersten Element hineinfährt. Dadurch wird sichergestellt, dass der freiliegende Fadenabschnitt an die durch die Öffnung definierte Übergabestation gebracht wird, so dass er von dem Fadenfänger ergriffen werden kann.

In einer weiteren Ausgestaltung der Erfindung weist der Fadenfänger einen Fangschlitz zur Aufnahme des Fadens auf.

Diese Maßnahme hat den Vorteil, dass der Faden, nachdem er von dem Fadenfänger ergriffen ist, beim Abziehen des Fadens über das erste Element in dem Fangschlitz einen sicheren Sitz hat. Somit kann der Faden abgezogen werden, ohne dass die Gefahr besteht, dass er sich von dem Fadenfänger löst.

In einer weiteren Ausgestaltung der Erfindung ist der Fadenfänger mit einer Spitze versehen, deren Endpunkt, bezogen auf eine Mittellängsachse des Hohlraumes in dem ersten Element, seitlich versetzt ist.

Dies führt zu dem Vorteil, dass die seitlich versetzte Spitze des Fadenfängers beim Ergreifen des Fadens an dem freiliegenden Fadenabschnitt seitlich vorbei läuft. Dadurch wird sichergestellt, dass die Spitze den Faden nicht trifft und somit der Faden von der Spitze nicht beeinträchtigt wird.

Weitere Merkmale und Vorteile ergeben sich aus der nachfolgenden Beschreibung und der beigefügten Zeichnung.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand ausgewählter Ausführungsbeispiele in Zusammenhang mit den beiliegenden Zeichnungen näher beschrieben und erläutert.

Es zeigen:
- Fig. 1: eine Seitenansicht einer erfindungsgemäßen Vorrichtung in einer Ausgangsstellung;
- Fig. 2: eine der Darstellung in Fig. 1 vergleichbare Darstellung, nachdem die beiden Elemente sich getroffen haben;
- Fig. 3: eine vergrößerte Darstellung eines distalen Abschnitts eines ersten Elements und eines zweiten Elements, und eine Seitenansicht eines Fadenfängers;
- Fig. 4: eine vergrößerte, teilweise geschnittene Darstellung, bei der der distale Abschnitt des zweiten Elements in eine Öffnung in dem ersten Element eingefahren ist;
- Fig. 5: eine vergrößerte, teilweise geschnittene Seitenansicht, nachdem die beiden Elemente sich getroffen haben, wobei der Fadenfänger in das erste Element eingeführt ist;
- Fig. 6: eine der Darstellung in Fig. 5 vergleichbare Darstellung, wobei der Fadenfänger einen freiliegenden Fadenabschnitt des Fadens trifft;
- Fig. 7: eine der Darstellung in Fig. 5 vergleichbare Darstellung, wobei der Faden in einem Fangschlitz des Fadenfängers fixiert ist;
- Fig. 8: eine Situation beim Einbringen eines Kanals in einem Knochen mit der erfindungsgemäßen Vorrichtung, wobei das erste Element in den Knochen eingetrieben ist; und
- Fig. 9: eine der Darstellung in Fig. 8 vergleichbare Darstellung, wobei das zweite Element in den Knochen eingetrieben ist und der Faden von dem Fadenfänger über das erste Element abgezogen ist.

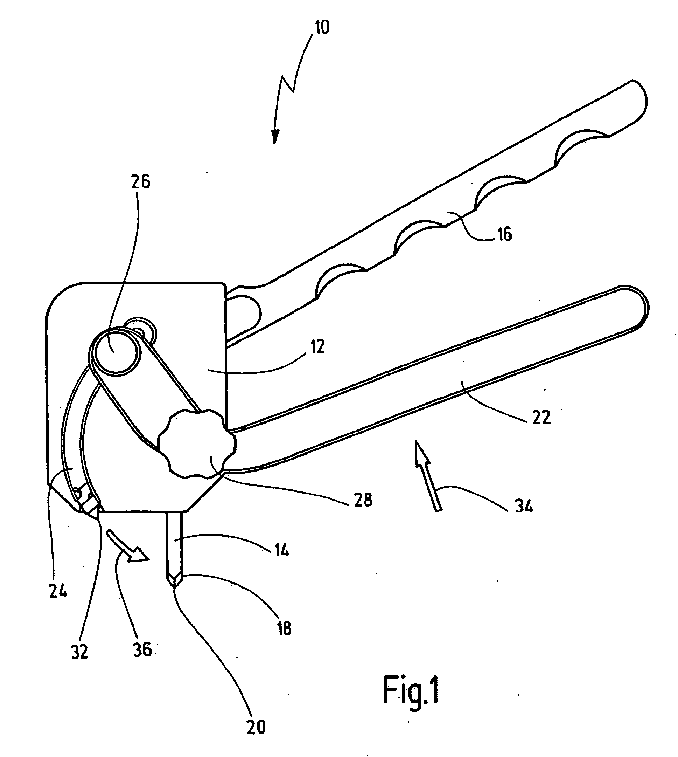

Eine in Fig. 1 bis 9 dargestellte Vorrichtung zum Befestigen eines chirurgischen Fadens an einem Knochen ist in ihrer Gesamtheit mit der Bezugsziffer 10 versehen.

Die erfindungsgemäße Vorrichtung 10 weist ein Gehäuse 12 auf, mit dem ein erstes Element 14 und ein Griff 16 fest verbunden sind.

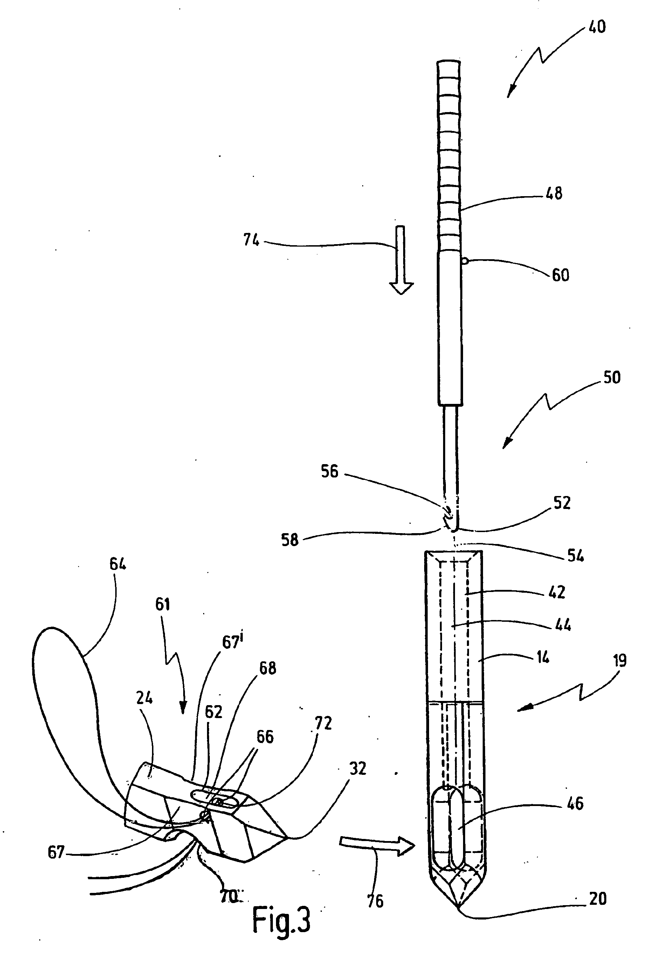

Das erste Element 14 ist unbewegbar und stabförmig ausgebildet. Ferner ist das erste Element 14 hohl ausgebildet, um einen Fadenfänger aufzunehmen, wie das später noch näher in Zusammenhang mit Fig. 3 beschrieben wird.

Ein distales Ende 18 des ersten Elements 14 ist als Spitze 20 ausgebildet, so dass das stabförmige Element 14 einfach und wenig traumatisch in einen Knochen eingetrieben werden kann.

Ferner weist die erfindungsgemäße Vorrichtung 10 einen beweglichen Hebel 22 auf, an dem ein zweites, bewegliches Element 24 mittels eines Zapfens 26 angebracht ist. Der bewegliche Hebel 22 ist mittels einer Mutter 28 an dem Gehäuse 12 befestigt. Durch Lösen der Mutter 28 kann der Hebel 22 mit dem an dem Hebel 22 angebrachten zweiten Element 24 von dem Gehäuse 12 abgenommen werden, so dass die Vorrichtung 10 gründlich gereinigt werden kann.

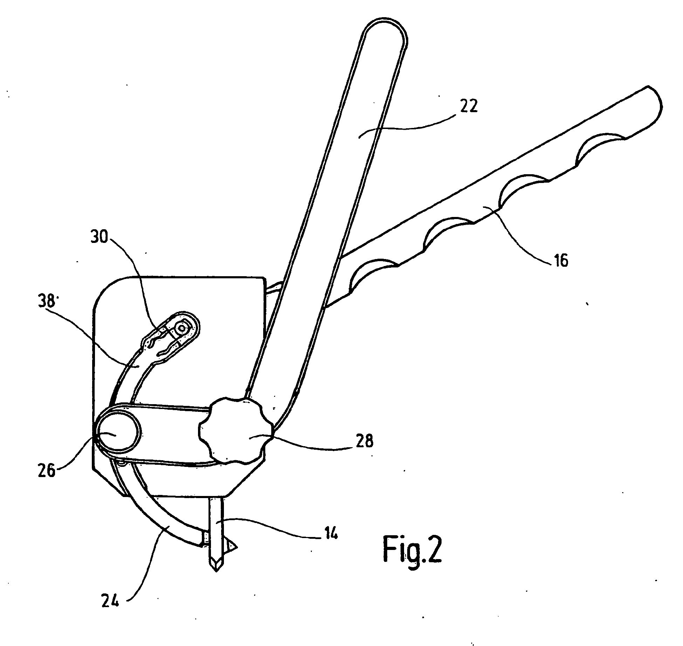

Der Hebel 22 wird in der aus Fig. 1 ersichtlichen Ausgangsposition über eine Klemme 30 gehalten, die aus der Darstellung in Fig. 2 ersichtlich ist.

Das zweite Element 24 ist gekrümmt ausgebildet und weist an seinem distalen Ende eine Spitze 32 auf, die das Eintreiben des Elements 24 in den Knochen vereinfacht.

Um die erfindungsgemäße Vorrichtung aus der in Fig. 1 dargestellten Ausgangsposition in zweite in Fig. 2 dargestellte Position zu bringen, wird der bewegliche Hebel 22 in eine durch einen Pfeil 34 angezeigte Richtung gezogen. Die Bewegung des Hebels 22 bewirkt eine Bewegung des mit dem Hebel 22 verbundenen zweiten Elements 24, wie es durch einen Pfeil 36 angezeigt ist. Das zweite Element 24 wird durch das Ziehen des Hebels 22 längs einer in dem Gehäuse 12 ausgebildeten Führungsbahn 38 bewegt, die den Weg des zweiten Elements 24 vorgibt. Die Führungsbahn 38 ist derart ausgerichtet, dass das zweite Element 24 das erste Element 14 trifft. Solch eine Situation geht aus Fig. 2 hervor.

In Fig. 3 ist eine vergrößerte Darstellung eines distalen Abschnitts 19 des ersten Elements 14 und eines distalen Abschnitts 61 des zweiten Elements 24 dargestellt. Ferner ist ein Fadenfänger 40 ersichtlich.

Aus dieser Figur geht hervor, dass das erste Element 14 einen Hohlraum 42 zur Aufnahme des Fadenfängers 40 aufweist. Der Hohlraum 42 ist als ein sich in axialer Richtung erstreckender Kanal 44 ausgebildet. Der Kanal 44 mündet an seinem distalen Ende in eine Langloch-Öffnung 46, die an dem distalen Abschnitt 19 des ersten Elements 14 angeordnet ist, so dass der Fadenfänger 40 durch den Kanal 44 in die Öffnung 46 hineinfahren kann.

Aus Fig. 3 ist ebenfalls der distale Abschnitt 61 des zweiten Elements 24 ersichtlich, der einen Faden 64 trägt.

Der distale Abschnitt 61 weist eine Bohrung 66 auf, die zum Durchführen des Fadens 64 dient. Unten weist der distale Abschnitt 61 des zweiten Elements 24 eine Einbuchtung 70 auf. Die Einbuchtung 70 dient dazu, dass der durch die Bohrung 66 gefädelte Faden 64 wieder rückgeführt werden kann.

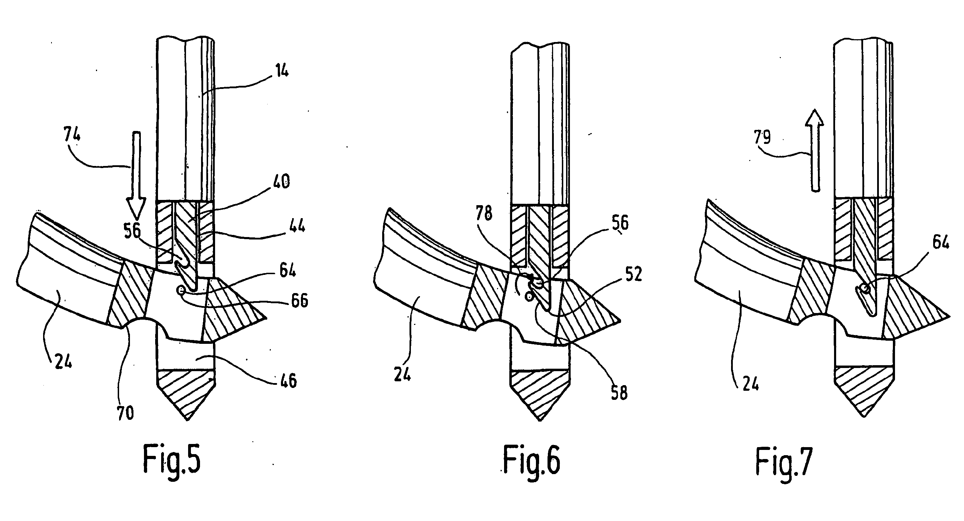

Ferner weist der distale Abschnitt 61 des zweiten Elements 24 eine Ausnehmung 62 auf, über die ein freiliegender Fadenabschnitt 72 des Fadens 64 von dem Fadenfänger 40 ergriffen werden kann, wie das noch später näher in Zusammenhang mit Fig. 5 bis 7 beschrieben wird.

Die Ausnehmung 62 ist als Langloch 68 ausgebildet.

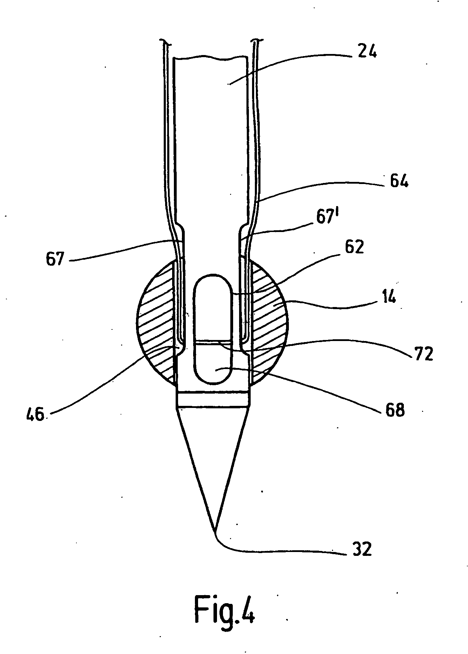

Die Bohrung 66, durch die der Faden 64 durchgeführt wird, schneidet die Ausnehmung 62 etwa mittig. Somit ist der freiliegende Fadenabschnitt 72 des Fadens 64 quer zu der Ausnehmung 62 angeordnet, wie es insbesondere aus der Darstellung von Fig. 4 hervorgeht.

Beidseits der Bohrung 66 sind äußere Einbuchtungen 67, 67' vorhanden, die Platz für den Faden 64 schaffen, damit dieser durch die Kanten der Öffnung 46 im distalen Abschnitt 19 des ersten Elementes 14 nicht beeinträchtigt wird.

Aus Fig. 3 ist ebenso der Fadenfänger 40 ersichtlich, der einen stabförmigen Körper 48 aufweist. Der Körper 48 weist einen distalen Abschnitt 50 auf, der einen kleineren Durchmesser aufweist als der restliche Körper 48. Der Durchmesser des Körpers 48 des Fadenfängers 40 ist dem als Kanal 44 ausgebildeten Hohlraum 42 in dem ersten Element 14 angepasst.

Der Fadenfänger 40 weist ferner eine Spitze 52 auf, die bezogen auf eine Mittellängsachse 54 des Hohlraumes 42 in dem ersten Element 14 seitlich versetzt ist, wobei die Spitze 52 so weit seitlich versetzt ist, dass die Spitze 52 nahe der Wand des Hohlraums 42 zum Liegen kommt.

Ferner ist der distale Abschnitt 50 des Fadenfängers 40 mit einem schrägen Fangschlitz 56 versehen, der zur Aufnahme des Fadens 64 dient. Der Fangschlitz 56 ist an seinem der Spitze 52 abgewandten Ende offen, so dass der von dem Fadenfänger 40 ergriffene Faden 64 beim Abziehen über das erste Element 14 in dem Fangschlitz 56 sicher fixiert ist.

Ferner weist die Spitze 52 eine geneigte Flanke 58 auf, so dass der Faden 64 entlang der Flanke 58 in den Fangschlitz 56 gleiten kann.

Der Fadenfänger 40 weist an dem Körper 48 einen Stift 60 auf. Der Stift 60 gibt die Position vor, in der der Fadenfänger 40 in den Hohlraum 42 des ersten Elements 14 eingeführt werden kann.

Wie schon oben beschrieben wurde, wird das zweite Element 24 durch Ziehen des Hebels 22 in eine durch einen Pfeil 76 angezeigte Richtung entlang der Führungsbahn 38 so lange bewegt, bis der den Faden 64 tragende distale Abschnitt 61 des zweiten Elements 24 in die Öffnung 46 in dem ersten Element 14 hineinfährt.

Nachdem die beiden Elemente 14, 24 sich getroffen haben, d.h. wenn der distale Abschnitt 61 des zweiten Elements 24 in die Öffnung 46 eingefahren ist, kann der freiliegende Fadenabschnitt 72 des Fadens 64 von dem Fadenfänger 40 ergriffen werden, wie das jetzt anhand Fig. 5 bis 7 beschrieben wird.

Um den Faden 64 zu ergreifen, wird der Fadenfänger 40, nachdem der distale Abschnitt 61 des zweiten Elements 24 in die Öffnung 46 in dem ersten Element eingefahren ist, in den Kanal 44 in dem ersten Element 14 eingeführt (s. Pfeil 74). Es ist anzumerken, dass der Fadenfänger 40 in einer durch den Stift 60 vorbestimmten Position in den Kanal 44 einbringbar ist. Dann wird der Fadenfänger so weit axial verschoben bis die Flanke 58 an den freiliegenden Fadenabschnitt 72 des Fadens 64 trifft, wie es aus Fig. 6 hervorgeht.

Dadurch, dass die Spitze 52 des Fadenfängers 40 seitlich versetzt ist, trifft sie den Faden 64 nicht, sondern läuft sie seitlich vorbei. Dadurch wird sichergestellt, dass der Faden 64 nicht beeinträchtigt wird.

Durch weiteres Verschieben des Fadenfängers 40 gleitet der Faden 64 längs der geneigten Flanke 58 und tritt in den Fangschlitz 56 ein (s. Pfeil 78). Dann ist der Faden 64 im Fangschlitz 56 gefangen. Solch eine Situation ist in Fig. 7 dargestellt.

Nachdem der Faden 64 in dem Fangschlitz 56 gefangen ist, wird der Fadenfänger 40 mit dem Faden 64 über das erste Element 14 abgezogen (s. Pfeil 79).

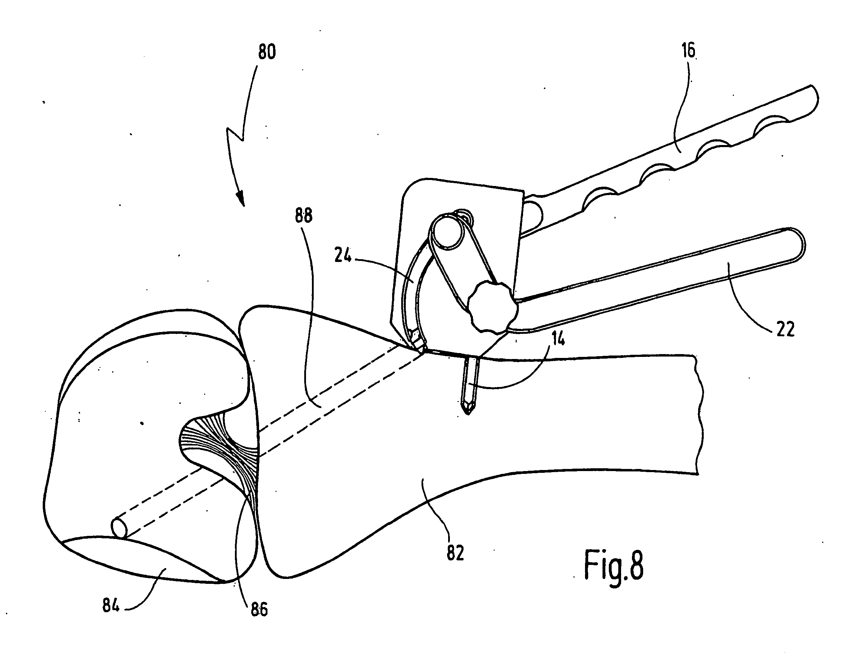

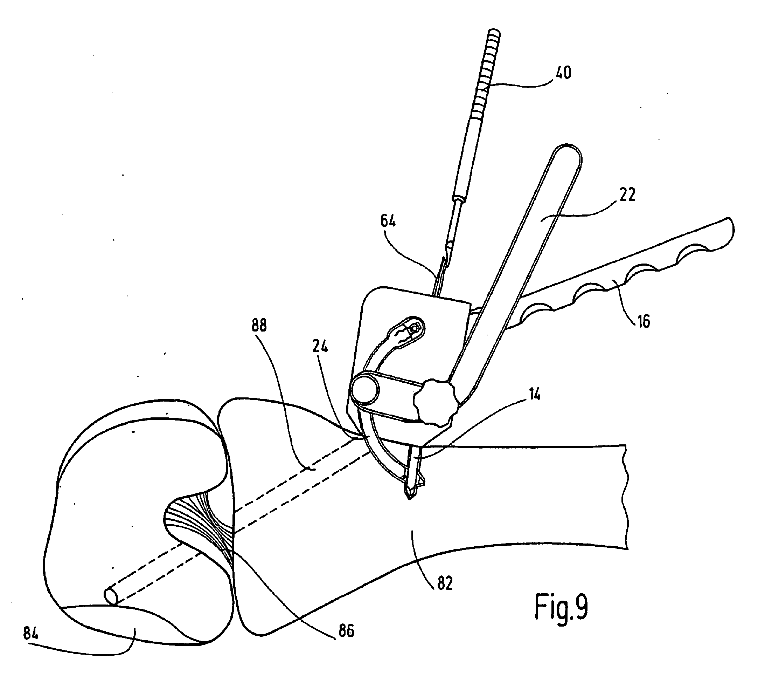

Anhand Fig. 8 und 9 soll eine mögliche Handhabung der erfindungsgemäßen Vorrichtung 10 kurz erläutert werden.

In Fig. 8 ist ein menschliches Kniegelenk 80 stark schematisiert dargestellt.

Das Kniegelenk 80 ist ein Verbindungsgelenk zwischen der Tibia (Unterschenkelknochen) 82 und dem Femur (Oberschenkelknochen) 84. Der Oberschenkel ist aus der Bildfläche nach hinten abgewinkelt. Im Zentrum des menschlichen Knies liegen zwei sich überkreuzende Bänder, die von der Tibia 82 zum Femur 84 verlaufen, nämlich das hintere Kreuzband 86, und das vordere Kreuzband, das hier nicht ersichtlich ist, und das durch ein Implantat ersetzt werden soll.

Bei einer Rekonstruktion des abgerissenen vorderen Kreuzbands wird in eine im Knochen vorgebohrte Bohrung 88 ein Implantat, das hier nicht dargestellt ist, eingeführt und an dem entsprechenden Knochen oder an dem Körper befestigt.

Bei diesem in Fig. 8 dargestellten chirurgischen Vorgang soll ein hier nicht dargestellter das Implantat haltender Faden 64 an der Tibia 82 befestigt werden.

Dazu wird die erfindungsgemäße Vorrichtung 10 verwendet. Zuerst wird der distale Abschnitt 61 des zweiten Elements 24 mit dem Faden 64 des Implantates bestückt, und zwar so wie das aus Fig. 3 hervorgeht. In der Ausgangsposition wird die erfindungsgemäße Vorrichtung 10 an der Tibia 82 derart angesetzt, dass die Spitze 32 des zweiten Element 24 an dem vorhandenen tibialen Bohrkanal 88 angesetzt wird.

Danach wird das erste Element 14 in die Tibia 82 eingetrieben. Der bewegliche Hebel 22 wird verschwenkt, wodurch das gekrümmte Element 24 in die Tibia 82 eingetrieben wird.

Nachdem die Elemente 14, 24 sich getroffen haben, wird der Fadenfänger 40 in den als Kanal 44 ausgebildeten Hohlraum 42 in dem ersten Element 14 eingeführt, um den freiliegenden Fadenabschnitt 72 des von dem zweiten Element 24 getragenen Fadens 64 zu ergreifen. Das Ergreifen des Fadens 64 erfolgt genauso wie das in Fig. 5 bis 7 beschrieben wurde.

Danach wird der Fadenfänger 40 mit dem Faden 64 über das erste Element 14 abgezogen. Solch eine Situation ist in Fig. 9 dargestellt.

Zuletzt wird das zweite Element 24 durch Bewegung des beweglichen Hebels 22 in die Ausgangsposition gebracht, und dann wird das erste Element 14 aus der Tibia 82 zurückgezogen.

Mit dem an der Tibia 82 befestigten Faden 64 kann der hier nicht dargestellte das Implantat haltende Faden an der Tibia 82 fixiert werden.

## Patentansprüche

1. Vorrichtung zum Befestigen eines chirurgischen Fadens (64) an einem Knochen (82), mit einem ersten Element (14), das in einen Knochen (82) einbringbar ist, und mit einem zweiten bewegbaren Element (24), das derart in den Knochen (82) einbringbar ist, das es das erste Element (14) trifft, wodurch ein Kanal in dem Knochen (82) bewerkstelligt wird, wobei eines der Elemente (24) einen chirurgischen Faden (64) derart trägt, dass der Faden (64), nachdem die Elemente (14, 24) sich getroffen haben, ergreifbar ist, **dadurch gekennzeichnet, dass** in dem Element (14), das keinen Faden (64) trägt, ein separater Fadenfänger (40) angeordnet ist, mittels dem der Faden (64), nachdem die Elemente (14, 24) sich getroffen haben, ergreifbar und über dieses Element (14) abziehbar ist, wobei die beiden Elemente (14, 24) derart ausgestaltet sind, dass sie beim Abziehen des Fadens (64) im Knochen (82) in der Lage bleiben, in der sie sich getroffen haben.

2. Vorrichtung nach Anspruch 1, **dadurch gekennzeichnet, dass** das erste Element (14) unbewegbar und stabförmig ausgebildet ist.

3. Vorrichtung nach Anspruch 1 oder 2, **dadurch gekennzeichnet, dass** ein distales Ende (18) des ersten Elements (14) als Spitze (20) ausgebildet ist.

4. Vorrichtung nach Anspruch 2 oder 3, **dadurch gekennzeichnet, dass** das erste Element (14) stabförmig ist, und dass der Fadenfänger (40) darin angeordnet ist.

5. Vorrichtung nach einem der Ansprüche 1 bis 4, **dadurch gekennzeichnet, dass** ein distaler Abschnitt (19) des ersten Elements (14) mit einer Öffnung (46) versehen ist.

6. Vorrichtung nach Anspruch 5, **dadurch gekennzeichnet, dass** ein distaler, den Faden (64) tragender Abschnitt (61) des zweiten Elements (24) in die Öffnung (46) in dem ersten Element (14) einfahrbar ist.

7. Vorrichtung nach Anspruch 6, **dadurch gekennzeichnet, dass** das zweite Element (24) an seinem distalen Abschnitt (61) den Faden (64) derart trägt, dass ein Fadenabschnitt (72) des Fadens (64) freiliegend ist, der nachdem die Elemente (14, 24) sich getroffen haben, von dem in dem ersten Element (14) angeordneten Fadenfänger (40) zwangsweise ergreifbar ist.

8. Vorrichtung nach einem der Ansprüche 5 bis 7, **dadurch gekennzeichnet, dass** der Fadenfänger (40) nur in einer definierten Position in das erste Element (14) einbringbar ist.

9. Vorrichtung nach Anspruch 7 oder 8, **dadurch gekennzeichnet, dass** in dem ersten Element (14) ein Hohlraum (42) zur Aufnahme des Fadenfängers (40) vorhanden ist.

10. Vorrichtung nach Anspruch 9, **dadurch gekennzeichnet, dass** der Hohlraum (42) an seinem distalen Ende in die Öffnung (46) in dem ersten Element (14) mündet.

11. Vorrichtung nach Anspruch 9 oder 10, **dadurch gekennzeichnet, dass** der den Faden (64) tragende distale Abschnitt (61) des zweiten Elements (24) eine Ausnehmung (62) aufweist, über die der freiliegende Fadenabschnitt (72) von dem Fadenfänger (40) ergreifbar ist.

12. Vorrichtung nach Anspruch 11, **dadurch gekennzeichnet, dass** der distale Abschnitt (61) des zweiten Elements (24) eine Bohrung (66) zum Durchführen des Fadens (64) aufweist, die die Ausnehmung (62) schneidet.

13. Vorrichtung nach Anspruch 11 oder 12, **dadurch gekenntzeichnet, dass** die Ausnehmung (62) als eine Nut ausgebildet ist.

14. Vorrichtung nach einem der Ansprüche 11 bis 13, **dadurch gekennzeichnet, dass** die Ausnehmung (62) als Langloch (68) ausgebildet ist.

15. Vorrichtung nach einem der Ansprüche 9 bis 14, **dadurch gekennzeichnet, dass** das zweite Element (24) gekrümmt ist.

16. Vorrichtung nach einem der Ansprüche 9 bis 15, **dadurch gekennzeichnet, dass** das zweite Element (24) an seinem distalen Ende eine Spitze (32) aufweist.

17. Vorrichtung nach einem der Ansprüche 9 bis 16, **dadurch gekennzeichnet, dass** das zweite Element (24) entlang einer Führungsbahn (38) führbar ist.

18. Vorrichtung nach einem der Ansprüche 9 bis 17, **dadurch gekennzeichnet, dass** der Fadenfänger (40) einen Fangschlitz (56) zur Aufnahme des Fadens (64) aufweist.

19. Vorrichtung nach einem der Ansprüche 9 bis 18, **dadurch gekennzeichnet, dass** der Fadenfänger (40) mit einer Spitze (52) versehen ist, deren Endpunkt, bezogen auf eine Mittellängsachse (54) des Hohlraumes (42) in dem ersten Element (14) seitlich versetzt ist.

## Claims

1. Device for securing a surgical suture (64) to a bone (82), with a first element (14), which can be introduced into a bone (82), and with a second, movable element (24), which can be introduced into the bone (82) in such a way that it meets the first element (14), as a result of which a channel is formed in the bone (82), with one of the elements (24) carrying a surgical suture (64) in such a way that the suture (64) can be taken hold of after the elements (14, 24) have met, **characterized in that** a separate suture catcher (40) is disposed in the element (14) not carrying a suture (64), by means of which the suture (64) can be taken hold of, after the elements (14, 24) have met, and can be withdrawn via this element (14), wherein both elements (14, 24) are designed in such a way that they remain in the bone (82) **in that** position in which they met, while the suture (64) is withdrawn.

2. Device according to claim 1, **characterized in that** the first element (14) is immovable and rod-shaped.

3. Device according to claim 1 or 2, **characterized in that** a distal end (18) of the first element (14) is designed as a tip (20).

4. Device according to claim 2 or 3, **characterized in that** the first element (14) is rod-shaped and that the suture catcher (40) is arranged therein.

5. Device according to one of claims 1 to 4, **characterized in that** a distal portion (19) of the first element (14) is provided with an opening (46).

6. Device according to claim 5, **characterized in that** a distal portion (61) of the second element (24) carrying the suture (64) can be driven into the opening (46) in the first element (14).

7. Device according to claim 6, **characterized in that** the second element (24) carries the suture (64) at its distal portion (61) in such a way that a suture portion (72) of the suture (64) is exposed and, after the elements (14, 24) have met, can be forcibly taken hold of by the suture catcher (40) arranged in the first element (14).

8. Device according to one of claims 5 to 7, **characterized in that** the suture catcher (40) can be introduced into the first element (14) only in a defined position.

9. Device according to claim 7 or 8, **characterized in that** a cavity (42) for receiving the suture catcher (40) is present in the first element (14).

10. Device according to claim 9, **characterized in that** cavity (42) opens at its distal end into the opening (46) in the first element (14).

11. Device according to claim 9 or 10, **characterized in that** the distal portion (61) of the second element (24) carrying the suture (64) comprises a recess (62) via which the exposed suture portion (72) can be taken hold of by the suture catcher (40).

12. Device according to claim 11, **characterized in that** the distal portion (61) of the second element (24) comprises a bore (66) for passing through the suture (64) which intersects the recess (62).

13. Device according to claim 11 or 12, **characterized in that** the recess (62) is designed as a groove.

14. Device according to one of claims 11 to 13, **characterized in that** the recess (62) is designed as an oblong hole (68).

15. Device according to one of claims 9 to 14, **characterized in that** the second element (24) is curved.

16. Device according to one of claims 9 to 15, **characterized in that** the second element (24) comprises a tip (32) at its distal end.

17. Device according to one of claims 9 to 16, **characterized in that** the second element (24) can be guided along a guide path (38).

18. Device according to one of claims 9 to 17, **characterized in that** the suture catcher (40) comprises a catching slit (56) for receiving the suture (64).

19. Device according to one of claims 9 to 18, **characterized in that** the suture catcher (40) is provided with a tip (52) whose end point is laterally offset with respect to a central longitudinal axis (54) of the cavity (42) in the first element (14).

## Revendications

1. Dispositif de fixation d'un fil chirurgical (64) sur un os (82), comprenant un premier élément (14) pouvant être introduit dans un os (82) et un second élément mobile (24) qui peut être introduit, dans ledit os (82), de manière qu'il vienne rencontrer ledit premier élément (14) en ménageant ainsi un canal, dans ledit os (82), l'un (24) desdits éléments portant un fil chirurgical (64) de façon telle que ledit fil (64) puisse être saisi après que lesdits éléments (14, 24) se sont rencontrés, **caractérisé par le fait que** l'élément (14), ne portant aucun fil (64), renferme un croche-fil distinct (40) au moyen duquel le fil (64) peut être saisi après rencontre mutuelle desdits éléments (14, 24), et peut être extrait par l'intermédiaire de cet élément (14), les deux éléments (14, 24) étant conçus pour conserver dans l'os (82), lors de l'extraction du fil (64), la position dans laquelle ils se sont rencontrés.

2. Dispositif selon la revendication 1, **caractérisé par le fait que** le premier élément (14) est de réalisation immobile et revêt la forme d'une tige.

3. Dispositif selon la revendication 1 ou 2, **caractérisé par le fait qu'**une extrémité distale (18) du premier élément (14) est réalisée sous la forme d'une pointe (20).

4. Dispositif selon la revendication 2 ou 3, **caractérisé par le fait que** le premier élément (14) revêt la forme d'une tige ; et **par le fait que** le croche-fil (40) est logé dans ledit élément.

5. Dispositif selon l'une des revendications 1 à 4, **caractérisé par le fait qu'**une région distale (19) du premier élément (14) est pourvue d'un orifice (46).

6. Dispositif selon la revendication 5, **caractérisé par le fait qu'**une région distale (61) du second élément (24), portant le fil (64), peut être insérée dans l'orifice (46) pratiqué dans le premier élément (14).

7. Dispositif selon la revendication 6, **caractérisé par le fait que** le second élément (24) porte le fil (64), dans sa région distale (61), de manière à dégager un segment (72) dudit fil (64) qui peut être saisi à force, par le croche-fil (40) logé dans le premier élément (14), après que lesdits éléments (14, 24) se sont rencontrés.

8. Dispositif selon l'une des revendications 5 à 7, **caractérisé par le fait que** le croche-fil (40) ne peut être introduit, dans le premier élément (14), qu'en un emplacement bien défini.

9. Dispositif selon la revendication 7 ou 8, **caractérisé par** la présence, dans le premier élément (14), d'une cavité (42) destinée à recevoir le croche-fil (40).

10. Dispositif selon la revendication 9, **caractérisé par le fait que** la cavité (42) débouche, par son extrémité distale, dans l'orifice (46) pratiqué dans le premier élément (14).

11. Dispositif selon la revendication 9 ou 10, **caractérisé par le fait que** la région distale (61) du second élément (24), portant le fil (64), présente un évidement (62) par l'intermédiaire duquel le segment (72) dudit fil, occupant une position dégagée, peut être saisi par le croche-fil (40).

12. Dispositif selon la revendication 11, **caractérisé par le fait que** la région distale (61) du second élément (24) offre un perçage (66) affecté au passage du fil (64) et traçant une intersection avec l'évidement (62).

13. Dispositif selon la revendication 11 ou 12, **caractérisé par le fait que** l'évidement (62) est réalisé sous la forme d'une rainure.

14. Dispositif selon l'une des revendications 11 à 13, **caractérisé par le fait que** l'évidement (62) est réalisé sous la forme d'un trou oblong (68).

15. Dispositif selon l'une des revendications 9 à 14, **caractérisé par le fait que** le second élément (24) est arqué.

16. Dispositif selon l'une des revendications 9 à 15, **caractérisé par le fait que** le second élément (24) comporte une pointe (32) à son extrémité distale.

17. Dispositif selon l'une des revendications 9 à 16, **caractérisé par le fait que** le second élément (24) peut être guidé le long d'une piste de guidage (38).

18. Dispositif selon l'une des revendications 9 à 17, **caractérisé par le fait que** le croche-fil (40) est muni d'une fente interceptrice (56) conçue pour recevoir le fil (64).

19. Dispositif selon l'une des revendications 9 à 18, **caractérisé par le fait que** le croche-fil (40) est doté d'un éperon (52) dont le point d'extrémité est décalé, dans le sens latéral, vis-à-vis d'un axe médian longitudinal (54) de la cavité (42) réservée dans le premier élément (14).
